(19) Europäisches Patentamt
European Patent Office
Office européen des brevets

(11) Numéro de publication: 0 004 495
B1

(12) # FASCICULE DE BREVET EUROPEEN

(21) Numéro de dépôt: 79400150.3

(22) Date de dépôt: 08.03.79

(51) Int. Cl.$^3$: C 07 C 55/06, C 07 C 51/58

(54) **Procédé de préparation de chlorure d'oxalyle**

(30) Priorité: 17.03.78 FR 7807740

(43) Date de publication de la demande:
03.10.79 Bulletin 79/20

(45) Mention de la délivrance du brevet:
10.12.80 Bulletin 80/25

(84) Etats contractants désignés:
BE DE FR GB IT

(56) Documents cités:
Néant

(73) Titulaire: RHONE-POULENC INDUSTRIES
22, avenue Montaigne
F-75008 Paris (FR)

(72) Inventeur: Acloque, André
26, rue Paul Cézanne
F-04600 Saint-Auban (FR)
Lanet, Jean-Claude
2, rue de la Colline
F-04600 Saint Auban (FR)
Correia, Yves
Les Lauzières
F-04160 Château Arnoux (FR)

(74) Mandataire: Cazes, Jean-Marie, et al
RHONE POULENC Service Brevets chimie et Polymères B.P. 753
F-75360 Paris Cedex 08/FR

EP 0 004 495 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Procédé de préparation de chlorure d'oxalyle

La présente invention a pour objet un procédé de préparation de chlorure d'oxalyle par chloroxydation de l'éthylèneglycol.

Il est connu d'après le brevet américain n° 2.816.140 de préparer du chlorure d'oxalyle par chauffage à une température comprise entre 35 et 135°C, d'un ester de tétrachloroéthylèneglycol et d'un acide carboxylique en présence d'un catalyseur constitué de charbon actif ou d'un dérivé d'un élément du groupe V A de la classification périodique (Fischer Periodic Chart—1951) et de poids atomique compris entre 7 et 33, dérivé tel que les amines tertiaires, les amides.

Ainsi, du chlorure d'oxalyle se forme simultanément à du chlorure de trichloracétyle par chauffage du bis trichloracétate de tétrachloroéthylèneglycol dans du chlorobenzène en présence de diméthylformamide comme catalyseur à une température comprise entre 100 et 120°C.

Ce même brevet indique que les esters de tétrachloroéthylèneglycol mis en oeuvre peuvent être obtenus par chloration des esters d'éthylèneglycol correspondants, chloration réalisée par voie photochimique avec du chlore en excès, à une température de 75—125°C, généralement en milieu solvant inerte tel que le tétrachlorure de carbone.

Un tel procédé présente l'inconvénient d'être réalisé en présence de solvants (tétrachlorure de carbone pour la chloration des esters d'éthylène glycol et chlorobenzène pour le réarrangement des esters de tétrachloroéthylèneglycol), solvants qu'il est nécessaire de séparer. De plus, l'opération de chloration en présence de tétrachlorure de carbone (Eb 77°C) ne peut être réalisée qu'à une température assez basse.

Le procédé de préparation de chlorure d'oxalyle, objet de la présente invention, consiste:

1) à faire réagir de l'éthylèneglycol sur du chlorure de trichloracétyle

2) à effectuer une chloration par voie photochimique du bis trichloracétate d'éthylèneglycol formé

3) à effectuer un réarrangement du bis trichloracétate de tétrachloroéthylèneglycol formé, en chlorure d'oxalyle et chlorure de trichloracétyle,

ledit procédé étant caractérisé en ce que les opérations 1 et 3 et éventuellement 2 sont réalisées en l'absence de tout solvant autre que le chlorure de trichloracétyle, et en ce que l'opération de chloration est réalisée à une température comprise entre 50 et 200°C, de préférence entre 80 et 160°C.

Le procédé objet de l'invention fait appel aux trois réactions suivantes:

$$(1)\quad 2\ CCl_3\ COCl + CH_2OH - CH_2OH \longrightarrow CCl_3COO\ CH_2 - CH_2OOC\ CCl_3 + 2HCl$$

$$(2)\quad CCl_3\ COOCH_2 - CH_2\ OOCCCl_3 + 4\ Cl_2 \longrightarrow CCl_3\ COO\ CCl_2 - CCl_2\ OOC\ CCl_3 + 4\ HCl$$

$$(3)\quad CCl_3\ COO\ CCl_2 - CCl_2 - OOC\ CCl_3 \longrightarrow 2\ CCl_3\ COCl + COCl - COCl$$

soit globalement

$$CH_2OH - CH_2OH + 4\ Cl_2 \longrightarrow CClO - CClO + 6\ HCl$$

La réaction globale revient donc à une chloroxydation de l'éthylèneglycol.

Pour une bonne réalisation du procédé, il est préférable d'éviter la présence de sels métalliques tels que des sels de fer, nickel, d'aluminium, de cobalt, de platine, en quantité supérieur à 1 ppm. Pour cela, il est préférable d'opérer dans des appareillages en verre, en acier revêtu ou en polymères exempts de charges métalliques.

Une contamination accidentelle des réactifs par des sels métalliques est plus facilement évitée lorsque l'on réalise les trois opérations du procédé dans un seul appareillage.

La réaction (1) et d'une manière générale la préparation d'esters par réaction des chlorures d'acide sur les alcools, sont décrites dans la littérature (Scattergood, Hershenson, J.A.C.S. *72,* (1950), 2808).

Pour une bonne réalisation de la réaction (1), on opère en présence d'un excè de 2 à 500% en poids de chlorure de trichloracétyle par rapport à la stoechiométrie, à une température comprise entre 60 et 80°C au

début de la réaction, puis on laisse monter la température jusqu'à la température de reflux du système.

La réaction (2) est réalisée en présence de tout rayonnement capable d'être absorbé par la molécule de chlore, c'est-à-dire tout rayonnement émettant entre 200 et 600 nanomètres environ.

A titre d'exemple, on peut citer: les lampes à vapeur de mercure basse, haute et moyenne pression possédant un revêtement permettant la réémission des raies de longueurs d'onde susceptibles d'être absorbées par le chlore, les lampes ordinaires à filament, les éclairages dits "au néon".

Pour une bonne réalisation de cette réaction, on opère de préférence à une pression comprise entre 500 mm Hg absolus et 5 bars, en présence d'un léger excès de chlore, de l'ordre de 1 à 5%, par rapport à la stoechiométrie, léger excès qui est entraîné par l'acide chlorhydrique gazeux formé.

Le chlore utilisé doit être sec, présenter une pureté de l'ordre de 99,8%, contenir de préférence moins de 0,1% d'oxygène et être exempt de traces de métaux, de fer en particulier sous forme de métal ou de sels.

La réaction (3) est réalisée à une température comprise entre 50 et 160°C, de préférence entre 60 et 120°C, en présence de chlorure de trichloracétyle et de 2 à 1000 ppm, de préférence de 20 à 250 ppm, d'un catalyseur tel qu'une amine tertiaire (triéthylamine, pyridine, diméthylaniline) ou son chlorhydrate.

Les exemples suivants sont donnés à titre indicatif et ne peuvent être considérés comme une limite du domaine et de l'esprit de l'invention.

## Exemple 1

L'appareillage utilisé est un appareillage en verre, constitué d'un ballon de 4 litres, équipé d'une agitation mécanique, d'un thermomètre, d'une ampoule à brome, surmonté d une colonne à distiller et Eclairé extérieurement par une lampe de 200 watts.

On introduit dans le ballon 15 moles de chlorure de trichloracétyle que l'on porte à 80—100°C et on ajoute en 2 heures 6 moles de glycol anhydre.

L'acide chlorhydrique, qui se dégage, traverse la colonne à distiller et est absorbé par de l'eau. On porte au reflux en soutirant l'excès de chlorure de trichloracétyle. Un dosage du fer à ce stade de la réaction révèle la présence de 0,1 mg/kg de fer dans le milieu.

On maintient alors le ballon à 130°C, et on introduit sous éclairement du chlore de manière à ce que l'acide chlorhydrique formé contienne environ 1% en poids de chlore; la température monte jusqu'à 160°C.

Au bout de 8 h 30, on a recueilli la quantité d'acide chlorhydrique correspondant à la stoechiométrie.

Le mélange réactionnel est refroidi en dégazant le chlore dissous par de l'azote, puis on ajoute l'excès de chlorure de trichloracétyle soutiré lors de l'opération d'estérification.

On ajoute au mélange, vers 60°C, 200 mg/kg de chlorhydrate de triéthylamine et on laisse monter progressivement la température sous agitation.

On recueille d'abord 618,5 g de chlorure d'oxalyle (Eb = 62°C) ce qui représente un rendement de 81% par rapport au glycol.

On recueille ensuite le chlorure de trichloracétyle (Eb = 120°C) avec un rendement de récupération de 90%, et enfin 164 g d'un résidu plus lourd ($Eb_{15}$=90—100°C) constitué par du bis (trichloracétate) de glycol partiellement chloré.

## Exemple 2

Cet exemple vise une variante du procédé décrit à l'exemple 1, consistant, après récupération par distillation du chlorure d'oxalyle formé, à ajuster la quantité de chlorure de trichloracétyle présente dans le ballon de manière à retrouver la même quantité de chlorure de trichloracétyle qu'au départ, et à procéder à un nouvel essai sans soutirer l'excès de chlorure de trichloracétyle après rectification; la chloration demande alors de 18 à 20 heures.

Cette variante du procédé permet d'améliorer le rendement en chlorure d'oxalyle (86% par rapport au glycol) sans modifier le taux de récupération de chlorure de trichloracétyle (90%).

## Exemples 3 à 6

On répète l'operation décrite à l'exemple 1 en effectuant la chloration à une température comprise entre 120 et 150°C, en présence de différentes quantités de fer, sous éclairement d'une lampe UV moyenne pression, l'opération de réarrangement étant réalisée en présence de 100 mg/kg de chlorhydrate de triéthylamine.

Les résultats de cette opération figurent dans le tableau suivant:

| Exemples | 3 | 4 | 5 | 6 |
|---|---|---|---|---|
| Durée de chloration en h | 32 | 26,5 | 31 | 28 |
| Rendement en COCl COCl | 48,5 | 38,5 | 72 | 84 |
| Rendement en $CCl_3$ COCl | 60 | 55 | 87 | 89 |
| Teneur en fer mg/kg | ~6 | ~2 | ~1 | <<1 |

Ces essais montrent l'influence néfaste de la présence de sels métalliques dans le milieu réactionnel.

Exemple 7

L'appareillage utilisé est en verre et est constitué d'un ballon de 4 litres, équipé d'un agitateur mécanique, d'un thermomètre, d'une ampoule à brome, surmonté d'une colonne à distiller et éclairé extérieurement par une lampe de 200 watts. On introduit dans le ballon 15 moles de chlorure de trichloracétyle que l'on porte à 80—100°C et on ajoute en 2 heures, 6 moles de glycol anhydre.

L'acide chlorhydrique qui se dégage traverse la colonne à distiller et est absorbée dans de l'eau; on porte ensuite le mélange pendant 1 heure au reflux. On ramène la température vers 80°C et on introduit du chlore sous éclairement en laissant la température monter progressivement jusqu' à 120°C; l'HCl qui s'échappe contient environ 1% de chlore. Au bout de 18 heures la quantité d'HCl sorti correspond à la quantité théorique. On refroidit alors à 60°C et on introduit 500 mg de chlorhydrate de triéthylamine et l'on laisse monter progressivement la température; en 2 heures, on arrive au reflux et on commence à soutirer le chlorure d'oxalyle formé.

On recueille 648 g de chlorure d'oxalyle, ce qui représente un rendement de 85% par rapport au glycol.

Le chlorure de trichloracétyle formé n'est pas distillé mais conservé dans le milieu réactionnel et réajusté en volume (8% de perte ont été constatés).

Exemple 8

Dans un réacteur en verre de 60 litres équipé d'une lampe à haute pression à mercure et possédant les dispositifs décrits à l'exemple 1, on introduit 285 moles de chlorure de trichloracétyle (51,87 kg).

Le milieu est porté à 80—100°C, et on introduit en 3 heures, 52,4 moles (3,249 kg) de glycol anhydre.

L'acide chlorhydrique, qui se dégage, traverse la colonne à distiller et est absorbé par de l'eau.

On porte au reflux pendant 2 heures, puis on ramène la température de la masse réactionnelle à 60°C; un dosage du fer à ce stade de la réaction, révèle la présence de moins de 0,1 mg/kg de fer dans le milieu.

On introduit alors sous éclairement pendant 24 heures, du chlore en excès de manière à ce que l'acide chlorhydrique sortant du réacteur contienne de 1 à 2% en poids de chlore, la température étant portée progressivement à 90°C puis 120°C.

On porte au reflux, on élimine le chlore par passage d'azote en fin de réaction.

On refroidit le milieu vers 60—65°C et on introduit 200 mg/kg de chlorhydrate de triéthylamine.

On laisse monter la température progressivement et on retire par distillation 5,66 kg de chlorure d'oxalyle (soit un rendement de 85% par rapport au glycol) et du chlorure de trichloracétyle avec un rendement de récupération de 96%.

Exemple 9

Dans un réacteur en acier émaillé de 1500 litres équipé d'une agitation d'une entrée de liquide et surmonté d'une colonne à distiller, on place 2 T ($11.10^{+3}$ moles) de chlorure de trichloracétyle de pureté 98% et on ajoute à 90°C environ en 2 heures 150 kg ($2{,}42.10^3$ moles) d'éthylèneglycol anhydre. On porte ensuite le mélange à 120°C pendant 1 heure. La solution de bis trichloracétate d'éthylèneglycol dans le chlorure de trichloracétyle contient moins de 1 ppm de fer. Le mélange réactionnel est introduit alors dans un réacteur de photochloration présentant un rapport surface d'éclairement/volume de 28 m2/m3 et équipé de lampe à vapeur de mercure réémettant de la lumière à plus de 400 nm. La température de départ etant d'environ 80°C on introduit du chlore de telle manière que l'acide chlorhydrique à la sortie n'en contienne pas plus de 1% en volume. Après 24 heures de chloration al température étant passée de 80 à 110°C, et le mélange ne présentant en IR aucune bande caractéristique de l'hydrogène, la chloration est arrêtée; on fait passer un courant d'azote de 10 m3/h pendant 1/4 d'heure pour chasser le chlore dissous et on transvase le milieu réactionnel dans un réacteur de 1500 litres en acier verré où l'on ajoute à 55—60°C 100 g de chlorhydrate de triéthylamine et on laisse monter progressivement la température en 2 heures pour atteindre le reflux.

On recueille 249 kg ($1{,}96.10^{+3}$ moles) de chlorure d'oxalyle (Eb = 62—64°C), ce qui

représente un rendement de 81% par rapport au glycol.

Le chlorure de trichloracétyle est conservé pour une nouvelle opération après réajustement de son volume.

## Revendications

1. Procédé de préparation de chlorure d'oxalyle, consistant:

(1)—à faire réagir de l'éthylèneglycol sur du chlorure de trichloracétyle,

(2)—à effectuer une chloration par voie photochimique du bistrichloracétate d'éthylèneglycol formé,

(3)—à effectuer un réarrangement du bistrichloracétate de tétrachloroéthylèneglycol formé, en chlorure d'oxyle et chlorure de trichloracétyle,

ledit procédé étant caractérisé en ce que les opérations (1) et (3) et éventuellement (2) sont réalisées en l'absence de tout solvant autre que le chlorure de trichloracétyle, et en ce que l'operation de chloration est réalisée à une température comprise entre 50 et 200°C.

2. Procédé selon la revendication 1 caractérisé en ce que l'opération de chloration est réalisée à une température comprise entre 80 et 160°C.

3. Procédé selon la revendication 1 ou la revendication 2 caractérisé en ce qu'il est réalisé en l'absence de sels métalliques en quantités supérieures à 1 ppm.

4. Procédé selon la revendication 1 ou la revendication 3 caractérisé en ce que l'étape d'estérification est réalisée en présence d'un excès de 2 à 500% en poids de chlorure de trichloracétyle par rapport à la stoechiométrie à une température croissant de 60 à 80°C jusqu'à la température de reflux du système.

5. Procédé selon les revendications 1, 2 ou 3 caractérisé en ce que l'opération de chloration est réalisée en présence d'un excès d'environ 1 à 5% de chlore par rapport à la stoechiométrie, sous une pression comprise entre 500 mm de mercure absolus et 5 bars.

6. Procédé selon la revendication 1 ou 1a revendication 3 caractérisé en ce que l'opération de réarrangement est réalisée à une température comprise entre 50 et 160°C en présence de 2 à 1000 ppm d'une amine tertiaire ou d'un chlorhydrate d'amine tertiaire comme catalyseur.

7. Procédé selon la revendication 6 caractérisé en ce que l'opération de réarrangement est réalisée à une température comprise entre 60 et 120°C en présence de 20 à 250 ppm d'amine tertiaire ou de chlorhydrate d'amine tertiaire.

8. Procédé selon la revendication 6 ou la revendication 7 caractérisé en ce que l'amine tertiaire est choisie parmi la triéthylamine, la pyridine, la diméthylaniline.

9. Procédé selon la revendication 1 et la revendication 3 caractérisé en ce que les 3 opérations sont réalisées dans un même appareillage.

10. Procédé selon la revendication 1 ou la revendication 3 caractérisé en ce que l'opération de chloration est réalisée dans un appareillage distinct de celui dans lequel sont réalisées les opérations d'estérification et de réarrangement.

11. Procédé selon la revendication 9 ou la revendication 10 caractérisé en ce que le ou les appareillages mis en oeuvre sont en verre, en acier ou en polymères exempts de charges métalliques.

## Claims

1. Process for the preparation of oxalyl chloride, which consists in:

(1) reacting ethylene glycol with trichloroacetyl chloride,

(2) photochemically chlorinating the ethylene glycol bis-trichloroacetate formed, and

(3) rearranging the tetrachloroethylene glycol bistrichloroacetate formed to give oxalyl chloride and trichloroacetyl chloride,

the said process being characterised in that the operations (1) and (3), and if appropriate (2), are carried out in the absence of any solvent other than the trichloroacetyl chloride, and in that the chlorination operation is carried out at a temperature between 50 and 200°C.

2. Process according to Claim 1, characterised in that the chlorination operation is carried out at a temperature between 80 and 160°C.

3. Process according to Claim 1 or Claim 2, characterised in that it is carried out in the absence of metal salts in amounts of more than 1 ppm.

4. Process according to Claim 1 or Claim 3, characterised in that the esterification step is carried out in the presence of an excess of 2 to 500% by weight of trichloroacetyl chloride, relative to stoichiometry, at a temperature increasing from 60 to 80°C up to the reflux temperature of the system.

5. Process according to Claims, 1, 2 or 3, characterised in that the chlorination operation is carried out in the presence of an excess of about 1 to 5% of chlorine, relative to stoichiometry, under an absolute pressure of between 500 mm of mercury and 5 bars.

6. Process according to Claim 1 or Claim 3, characterised in that the rearrangement operation is carried out at a temperature between 50 and 160°C, in the presence of 2 to 1,000 ppm of a tertiary amine or of a tertiary amine hydrochloride as the catalyst.

7. Process according to Claim 6, characterised in that the rearrangement operation is carried out at a temperature between 60 and 120°C, in the presence of 20 to 250 ppm of tertiary amine or of tertiary amine hydrochloride.

8. Process according to Claim 6 or Claim 7, characterised in that the tertiary amine is

chosen from amongst triethylamine, pyridine and dimethylaniline.

9. Process according to Claim 1 and Claim 3, characterised in that the 3 operations are carried out in one and the same apparatus.

10. Process according to Claim 1 or Claim 3, characterised in that the chlorination operation is carried out in a separate apparatus from that in which the esterification and rearrangement operations are carried out.

11. Process according to Claim 9 or Claim 10, characterised in that the apparatus or apparatuses employed are made of glass, steel or polymers which do not contain metal fillers.

**Patentansprüche**

1. Verfahren zur herstellung von Oxalylchlorid, bei dem man

(1) Äthylenglykol mit Trichloracetylchlorid umsetzt,

(2) eine Chlorierung des gebildeten Äthylenglykolbistrichloracetats auf photochemischem Wege vornimmt und

(3) eine Umlagerung des gebildeten Tetrachloräthylenglykolbistrichloracetats zu Oxalylchlorid und Trichloracetylchlorid vornimmt,

dadurch gekennzeichnet, daß man die Stufen (1) und (3) und gegebenenfalls (2) in Abwesenheit aller anderen Lösungsmittel als Trichloracetylchlorid durchführt und die Chlorierung bei einer Temperatur zwischen 50 und 200°C vornimmt.

2. Verfahren nach Anspruch 1, dadurch gekennzeichnet, daß man die Chlorierung bei einer Temperatur zwischen 80 und 160°C durchführt.

3. Verfahren nach Anspruch 1 oder 2, dadurch gekennzeichnet, daß man in Abwesenheit von Metallsalzen in mengen über 1 ppm arbeitet.

4. Verfahren nach Anspruch 1 oder 3, dadurch gekennzeichnet, daß man die Veresterung in Gegenwart eines Überschusses von 2 bis 500 Gewichtsprozent Trichloracetylchlorid, bezogen auf die stöchiometrische Menge, bei einer von 60 bis 80°C bis zur Rückflußtemperatur des Systems an steigender Temperatur durchführt.

5. Verfahren nach Anspruch 1, 2 oder 3, dadurch gekennzeichnet, daß mann die Chlorierung in Gegenwart eines Überschusses von etwa 1 bis 5% Chlor, bezogen auf die stöchiometrische Menge, unter einem Druck zwischen 500 mm Hg absolut und 5 bar durchführt.

6. Verfahren nach Anspruch 1 oder 3, dadurch gekennzeichnet, daß man die Umlagerung bei einer Temperatur zwischen 50 und 160°C in Gegenwart von 2 bis 1000 ppm eines tertiären Amins oder eines tertiären Aminhydrochlorids als Katalysator durchführt.

7. Verfahren nach Anspruch 6, dadurch gekennzeichnet, daß man die Umlagerung bei einer Temperatur zwischen 60 und 120°C in Gegenwart von 20 bis 250 ppm tertiärem Amin oder tertiärem Aminhydrochlorid durchführt.

8. Verfahren nach Anspruch 6 oder 7, dadurch gekennzeichnet, daß man als tertiäres Amin Triäthylamin, Pyridin und/oder Dimethylanilin verwendet.

9. Verfahren nach Anspruch 1 und 3, dadurch gekennzeichnet, daß man die drei Arbeitsschritte in der gleichen Apparatur durchführt.

10. Verfahren nach Anspruch 1 oder 3, dadurch gekennzeichnet, daß man die Chlorierung in einer Vorrichtung durchführt, die von der Vorrichtung, in der die Veresterung und die Umlagerung durchgeführt werden, getrennt ist.

11. Verfahren nach Anspruch 9 oder 10, dadurch gekennzeichnet, daß man Apparaturen verwendet, die aus Glas, Stahl oder Polymerisaten bestehen, die frei von metallischen Füllstoffen sind.